# EUROPEAN PATENT APPLICATION

(11) **EP 4 570 781 A1**
(43) Date of publication of application: **18.06.2025**
(21) Application number: 24747307.7
(22) Date of filing: 23.01.2024
(51) Int. Cl.: C07C 17/383, C07C 17/395, C07C 19/08, C07C 21/18

(54) **METHOD FOR PRODUCING PURIFIED TRANS-1,2-DIFLUOROETHYLENE (HFO-1132(E)) AND COMPOSITION CONTAINING HFO-1132(E)**

(30) Priority: 23.01.2023 JP 2023008172
(71) Applicant: DAIKIN INDUSTRIES, LTD., Osaka-shi, Osaka 530-0001 (JP)
(72) Inventor: TAKAHASHI, Kazuhiro, Osaka-Shi, Osaka 530-0001 (JP); NOGUCHI, Atsushi, Osaka-Shi, Osaka 530-0001 (JP); HASUMOTO, Yuuta, Osaka-Shi, Osaka 530-0001 (JP); TABUCHI, Akikazu, Osaka-Shi, Osaka 530-0001 (JP)
(74) Representative: Hoffmann Eitle
(86) International application number: PCT/JP2024/001869
(87) International publication number: WO 2024/157980

(57) **Abstract**

The present disclosure provides a method for efficiently purifying HFO-1132(E). The present disclosure specifically provides a method for producing purified HFO-1132(E), including an extractive distillation step of bringing a composition containing 1,1,1-trifluoroethane (HFC-143a) and trans-1,2-difluoroethylene (HFO-1132(E)) into contact with a solvent containing an amine to obtain a composition having a reduced amount of HFC-143a compared with the original composition.

## Description

### Technical Field

The present disclosure relates to a method for producing purified trans-1,2-difluoroethylene (HFO-1132(E)) and a composition containing HFO-1132(E).

### Background Art

HFO-1132(E) is attracting attention as a refrigerant that can replace the greenhouse gases such as difluoromethane (HFC-32) and 1,1,1,2,2-pentafluoroethane (HFC-125) because of its low global warming potential (GWP).

PTL 1 relates to the present disclosure and discloses a method for producing purified HFO-1132(E) and/or HFO-1123, comprising an extractive distillation step of bringing an azeotropic composition or azeotrope-like composition comprising difluoromethane (HFC-32) and trans-1,2-difluoroethylene (HFO-1132(E)) and/or 1,1,2-trifluoroethylene (HFO-1123) into contact with an extraction solvent to obtain a composition in which HFC-32 is reduced from the azeotropic composition or the azeotrope-like composition.

### Citation List

### Patent Literature

PTL 1: WO2021/261189A

### Summary of Invention

### Technical Problem

An object of the present disclosure is to provide a method for efficiently purifying HFO-1132(E) and to provide a composition containing HFO-1132(E).

### Solution to Problem

The present disclosure includes, for example, the subject matter described in the following items.

### Item 1.

A method for producing purified HFO-1132(E), comprising an extractive distillation step of bringing a composition containing 1,1,1-trifluoroethane (HFC-143a) and trans-1,2-difluoroethylene (HFO-1132(E)) into contact with a solvent containing an amine to obtain a composition having a reduced amount of HFC-143a compared with the original composition.

### Item 2.

The method according to Item 1, further comprising a distillation step of distilling the composition obtained in the extractive distillation step to separate the composition into a composition containing HFO-1132(E) as a main component and a composition containing the solvent containing an amine as a main component.

### Item 3.

The method according to Item 1 or 2, wherein the extractive distillation step comprises extractive distillation performed under a pressure of 0.05 to 5 MPaG (gauge pressure) using a first distillation column.

### Item 4.

The method according to Item 2 or 3, wherein the distillation step comprises distillation performed under a pressure of 0.05 to 3 MPaG (gauge pressure) using a second distillation column.

### Item 5.

The method according to any one of Items 1 to 4, further comprising a solvent recovery step of recovering the solvent containing an amine used in the extractive distillation step, and recirculating the recovered solvent containing an amine to the extractive distillation step.

### Item 6.

The method according to any one of Items 1 to 5, wherein the amine is represented by formula NR¹R²R³ wherein R¹, R², and R³ may be the same or different, and represent a hydrogen atom or an optionally substituted C₁₋₃ hydrocarbon group, with the proviso that not all of R¹, R², and R³ are a hydrogen atom.

### Item 7.

The method according to any one of Items 1 to 6, wherein the amine has a standard boiling point of -10 to 160°C. Item 8.

The method according to any one of Items 1 to 7, wherein the amine is at least one member selected from the group consisting of monomethylamine, dimethylamine, trimethylamine, monoethylamine, diethylamine, triethylamine, mono-n-propylamine, di-n-propylamine, tri-n-propylamine, mono-isopropylamine, and di-isopropylamine.

### Item 9.

The method according to Item 1, further comprising a distillation step of distilling the composition obtained in the extractive distillation step to separate the composition into a composition containing HFO-1132(E) as a main component and a composition containing the solvent containing an amine as a main component,
wherein
the extractive distillation step comprises extractive distillation performed under a pressure of 0.05 to 5 MPaG (gauge pressure) using a first distillation column, and
the distillation step comprises distillation performed under a pressure of 0.05 to 3 MPaG (gauge pressure) using a second distillation column,
the method further comprising
a solvent recovery step of recovering the solvent containing an amine used in the extractive distillation step, and recirculating the recovered solvent containing an amine to the extractive distillation step,
   wherein
the amine is at least one member selected from the group consisting of monomethylamine, dimethylamine, trimethylamine, monoethylamine, diethylamine, triethylamine, mono-n-propylamine, di-n-propylamine, tri-n-propylamine, mono-isopropylamine, and di-isopropylamine.

### Item 10.

A composition comprising trans-1,2-difluoroethylene (HFO-1132(E)), 1,1,1-trifluoroethane (HFC-143a), and an amine, wherein
the total concentration of the three components is 99.5 mass% or more based on the entire composition,
the content of HFC143a is more than 0 mass% and 0.4 mass% or less based on the entire composition, and
the content of the amine is more than 0 mass% and 0.1 mass% or less based on the entire composition.

### Item 11.

A composition comprising trans-1,2-difluoroethylene (HFO-1132(E)) and an amine,
wherein
the total concentration of the two components is 99.5 mass% or more based on the entire composition, and
the content of the amine is more than 0 mass% and 0.1 mass% or less based on the entire composition.

### Advantageous Effects of Invention

The present disclosure enables efficient purification of HFO-1132 (E) .

### Brief Description of Drawings

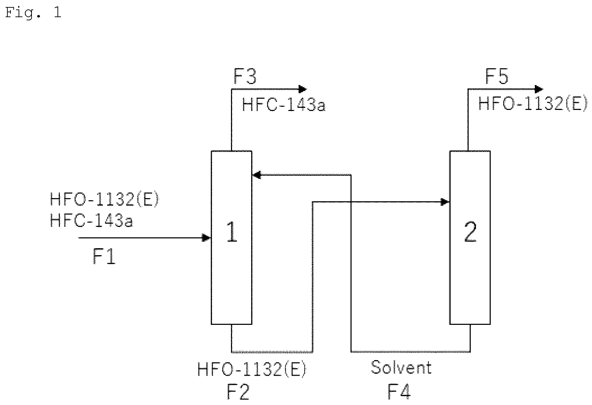

Fig. 1 shows an outline of the process in Example 1.

### Description of Embodiments

Extensive research was conducted, and it was found that HFO-1132(E) can be efficiently purified by a method including the extractive distillation step of bringing a composition containing HFC-143a and HFO-1132(E) into contact with a solvent containing an amine to obtain a composition having a reduced amount of HFC-143a compared with the original composition.

The present disclosure has been completed as a result of further research based on this finding.

In the present specification, a numerical range expressed using "to" refers to a range that includes the numerical values described before and after "to" as the lower and upper limits (i.e., "or more" and "or less").

In the present specification, an azeotropic composition refers to a composition that behaves as if it were a single substance with no difference in composition between the liquid phase and the gas phase under constant pressure.

In the present specification, an azeotrope-like composition refers to a composition that can form an azeotropic composition, and that has a composition close to an azeotropic composition and behavior like an azeotropic composition. An azeotrope-like composition can be distilled and/or refluxed with little change in composition. Therefore, an azeotrope-like composition can be treated almost the same as an azeotropic composition. One characteristic of azeotrope-like compositions is that the difference in pressure between the boiling point curve and the dew point curve in a pressure-composition diagram is within 5%.

In the present specification, the standard boiling point refers to the boiling point at a standard atmospheric pressure of 1013.25 hPa.

In the present specification, the gauge pressure refers to a relative pressure based on the atmospheric pressure, meaning the pressure difference obtained by subtracting the atmospheric pressure from the absolute pressure. In the present specification, the gauge pressure is denoted with "G" like, for example, MPaG. If "G" is not appended, the pressure is atmospheric pressure.

In the present specification, the "purity" of a refrigerant refers to the component ratio (mol% or mass%) determined by quantitative analysis using gas chromatography.

In the present specification, the main component refers to a component preferably contained in an amount of 85 mol% to 99.9 mol%, more preferably 90 mol% to 99.9 mol%, even more preferably 95 mol% to 99.9 mol%, and particularly preferably 99 mol% to 99.9 mol%.

In the present specification, extractive distillation refers to a distillation operation to add an extraction solvent to a mixture consisting of two or three components that have very similar standard boiling points and are difficult to separate by ordinary distillation, and having a specific volatility (relative volatility) close to 1, or a combination mixture with an azeotropic composition, to form an extraction mixture, and facilitate separation by adjusting the relative volatility of the original two or three components away from 1. When the relative volatility is 1, separation by distillation is impossible.

In the present specification, specific volatility (α) is defined as follows: if a composition containing at least component A of interest and component B of interest is in vapor-liquid equilibrium, the specific volatility of component A to component B is α_{A→B} = (y_{A}/x_{A})/(y_{B}/x_{B})
wherein
x_{A} represents the mole fraction of liquid-phase component A,
x_{B} represents the mole fraction of liquid-phase component B,
y_{A} represents the mole fraction of gas-phase component A in equilibrium with liquid-phase,
y_{B} represents the mole fraction of gas-phase component B in equilibrium with liquid-phase.

In the present specification, if component A is HFC-143a, and component B is HFO-1132(E), the specific volatility of component A to component B (i.e., the specific volatility of HFC-143a to HFO-1132(E)) is denoted as α_{143a→1132(E)}.

The present disclosure includes the following embodiments.

The production method of the present disclosure is a method for producing purified HFO-1132(E) and includes an extractive distillation step of bringing a composition containing HFC-143a and HFO-1132(E) into contact with a solvent containing an amine to obtain a composition having a reduced amount of HFC-143a compared with the original composition. In particular, it is preferable to apply the production method of the present disclosure when the composition containing HFC-143a and HFO-1132 (E) ("extractive distillation composition") is an azeotropic composition or azeotrope-like composition.

### Extractive Distillation Step

The extractive distillation step in the production method of the present disclosure is a step of bringing a composition containing HFC-143a and HFO-1132 (E) (extractive distillation composition) into contact with a solvent containing an amine to obtain a composition having a reduced amount of HFC-143a compared with the original composition. In other words, the extractive distillation step is a step of subjecting a composition containing HFC-143a and HFO-1132 (E) (extractive distillation composition) to extractive distillation in the presence of a solvent containing an amine to obtain a composition having a reduced amount of HFC-143a compared with the original composition. In the present disclosure, the term "reduce" in the extractive distillation step means to reduce the content of a specific compound (HFC-143a) in the extractive distillation composition.

The extractive distillation composition contains HFC-143a and HFO-1132 (E) in a total concentration of preferably 99.5 mass% or more, more preferably 99.7 mass% or more, even more preferably 99.8 mass% or more, and still more preferably 99.9 mass% or more.

The extractive distillation composition for use is, for example, a composition containing HFC-143a and HFO-1132 (E) in the total concentrations described above (in particular, an azeotropic composition or azeotrope-like composition) wherein in the process of producing HFO-1132(E), readily separable byproducts are separated through a pre-separation step (e.g., any distillation step) as necessary.

Although the extractive distillation composition preferably consists of HFO-1132(E) and HFC-143a, the presence of unavoidable impurities is acceptable depending on the conditions for preparing the extractive distillation composition.

HFC-143a (standard boiling point: -47.2°C) and HFO-1132(E) (standard boiling point: -53.0°C) are azeotropic or azeotrope-like. The azeotropic composition or azeotrope-like composition of HFC-143a and HFO-1132(E) has a boiling point lower than the boiling point of HFC-143a and the boiling point of HFO-1132(E) (azeotropic composition under 0.1013 MPa: HFC-143a = 0.94, temperature: -53.1°C).

The extractive distillation composition supplied in the extractive distillation step contains HFO-1132(E) in a molar percentage of preferably 80% or more and 99.999% or less, and more preferably 90% or more and 99.999% or less.

The extractive distillation step is preferably a step of bringing the extractive distillation composition into contact with a solvent containing an amine to subject the composition to extractive distillation, thereby obtaining a composition that contains HFO-1132(E) and that does not substantially contain HFC-143a.

In the present specification, the phrase "not substantially contain HFC-143a" means that the content of HFC-143a in the composition obtained in the extractive distillation step is preferably less than 1 mass%, more preferably less than 0.5 mass%, and particularly preferably less than 0.1 mass%.

In the extractive distillation step, the extraction solvent for use is a solvent containing an amine.

The amine can be any liquid amine that can be an extraction solvent, and is preferably an amine represented by formula: NR¹R²R³
wherein R¹, R², and R³ may be the same or different, and represent a hydrogen atom or an optionally substituted C₁₋₃ hydrocarbon group, with the proviso that not all of R¹, R², and R³ are a hydrogen atom.

Although the solvent containing an amine in the present disclosure preferably consists of an amine (an amine alone or an amine mixture), the presence of unavoidable impurities is acceptable to the extent that the impurities do not affect the extractive distillation step. The phrase "a solvent containing an amine" means substantially an amine, and may also be simply referred to as "extraction solvent."

The amine preferably has a standard boiling point of -10 to 160°C.

The amine is preferably at least one member selected from the group consisting of monomethylamine, dimethylamine, trimethylamine, monoethylamine, diethylamine, triethylamine, mono-n-propylamine, di-n-propylamine, tri-n-propylamine, mono-isopropylamine, and di-isopropylamine. Table 1 shows the Cas number and the standard boiling point of these amines.

**Table 1**

| Substance | Cas No. | Standard Boiling Point (°C) |
|---|---|---|
| Monomethylamine | 74-89-5 | -6 |
| Dimethylamine | 124-40-3 | 7.0 |
| Trimethylamine | 75-50-3 | 2.9 |
| Monoethylamine | 75-04-7 | 38 |
| Diethylamine | 109-89-7 | 56 |
| Triethylamine | 121-44-8 | 90 |
| Mono-n-propylamine | 107-10-8 | 48 |
| Di-n-propylamine | 142-84-7 | 110 |
| Tri-n-propylamine | 102-69-2 | 156 |
| Mono-isopropylamine | 75-31-0 | 32 |
| Di-isopropylamine | 108-18-9 | 84 |

These amines may be used alone, or in a combination of two or more.

Regarding the temperature range of the standard boiling point in the extractive distillation step, the temperature difference may be of a degree that the extraction solvent can be separated from the compound to be separated in the extractive distillation step by simple distillation, stripping, or the like; i.e., the temperature difference is generally 20°C or more. However, if the standard boiling point is too high, the extraction solvent itself may be decomposed. Therefore, the standard boiling point of the extraction solvent is preferably 30 to 135°C, more preferably 35 to 120°C, even more preferably 40 to 100°C, and particularly preferably 50 to 90°C, from the viewpoint of efficiently performing extractive distillation.

The amount of the extraction solvent used in the extractive distillation step is preferably 1 mol equivalent or more and 30 mol equivalents or less, and more preferably 5 mol equivalents or more and 25 mol equivalents or less, per mol equivalent of the extractive distillation composition supplied to an extractive distillation column.

The concentration of HFC-143a in the extractive distillation composition in the extractive distillation step is preferably 15 mol% or less, and more preferably 10 mol% or less. The lower limit of the concentration of HFC-143a in the extractive distillation composition in the extractive distillation step is preferably 0.001 mol% or more, more preferably 0.01 mol% or more, and even more preferably 0.1 mol% or more.

The number of theoretical plates of the extractive distillation column used in the extractive distillation step is preferably 10 or more, and more preferably 20 or more. The number of theoretical plates of the extractive distillation column used in the extractive distillation step is preferably 60 or less, and more preferably 50 or less, from an economical viewpoint.

In the extractive distillation step, the extraction solvent is preferably supplied to an upper plate of the extractive distillation column. The extraction solvent used in the extractive distillation step is preferably the extraction solvent that is recovered and recirculated in the extraction solvent recovery step, described later.

In the extractive distillation step, the pressure under which extractive distillation is performed (the pressure of the first distillation column) is preferably 0.05 to 5 MPaG (gauge pressure). The lower limit of the pressure is preferably 0.05 MPaG, more preferably 0.1 MPaG, even more preferably 0.25 MPaG, and particularly preferably 0.5 MPaG. The upper limit of the pressure is preferably 5 MPaG, more preferably 4 MPaG, even more preferably 3 MPaG, and particularly preferably 2 MPaG.

The extractive distillation step can be performed by a discontinuous operation or a continuous operation. The step is preferably performed by a continuous operation from an industrial viewpoint. Further, extractive distillation can be repeated to thereby purify the distillate component to a high purity.

For distilling HFC-143a from the extractive distillation composition in the extractive distillation step, it is preferable to use an extraction solvent that makes the relative volatility (specific volatility) of HFC-143a to HFO-1132(E) 1.1 or more, and preferably 1.2 or more, upon addition of the extraction solvent. This increases the gas-phase mole fraction of HFC-143a to thereby increase HFC-143a in the gas phase, thus enabling the separation of HFC-143a from the top of the extractive distillation column; thus, the extraction solvent and HFO-1123 (E) are obtained from the bottom of the extractive distillation column.

### Extraction Solvent Recovery Step

The production method of the present disclosure preferably includes an extraction solvent recovery step of recovering the extraction solvent used in the extractive distillation step, and recirculating the recovered extraction solvent to the extractive distillation step.

The extraction solvent recovery step includes a distillation step of distilling the composition obtained in the extractive distillation step to separate the composition into a composition containing HFO-1132(E) as a main component and a composition containing an extraction solvent as a main component ("distillation step" below), and can be performed by recovering the extraction solvent from the composition containing the extraction solvent as a main component and recirculating the recovered extraction solvent to the extractive distillation step.

The number of theoretical plates of the solvent recovery column (second distillation column) used in the distillation step is preferably 5 or more, and more preferably 10 or more. The number of theoretical plates of the solvent recovery column is preferably 40 or less, and more preferably 30 or less, from an economical viewpoint.

In the distillation step, the pressure under which distillation is performed is preferably 0.05 to 3 MPaG (gauge pressure). The lower limit of the pressure is preferably 0.05 MPaG, and more preferably 0.1 MPaG. The upper limit of the pressure is preferably 3 MPaG, and more preferably 2.5 MPaG.

The distillation step enables the separation of the composition containing HFO-1132(E) as a main component from the top of the solvent recovery column; thus, the composition containing an extraction solvent as a main component is obtained from the bottom of the solvent recovery column.

The extraction solvent recovery step can be performed by recovering an extraction solvent from the composition containing the extraction solvent as a main component obtained from the bottom in the distillation step, and recirculating the extraction solvent to the extractive distillation step. The extraction solvent recovered from the bottom may optionally be further subjected to a separation step, such as rectification, to remove impurities before recirculating it to the extractive distillation step.

The distillation step can be performed by a discontinuous operation or a continuous operation; the step is preferably performed by a continuous operation from an industrial viewpoint.

The production method of the present disclosure preferably includes the extractive distillation step and the extraction solvent recovery step, and more preferably includes an optional pre-distillation step for increasing the purity of an extractive distillation composition, the extractive distillation step, and the extraction solvent recovery step.

### Composition Containing HFO-1132(E), HFC-143a, and Amine

The present disclosure encompasses a composition containing trans-1,2-difluoroethylene (HFO-1132(E)), 1,1,1-trifluoroethane (HFC-143a), and an amine ("refrigerant 1" below). The type of the amine is as described in the extraction solvent section above mentioned.

Refrigerant 1 is a composition containing HFO-1132(E), HFC-143a, and an amine, wherein the total concentration of the three components is 99.5 mass% or more based on the entire composition, the content of HFC143a is more than 0 mass% and 0.4 mass% or less based on the entire composition, and the content of the amine is more than 0 mass% and 0.1 mass% or less based on the entire composition. The phrase "more than 0 mass%" can also be described as "0.01 mass% or more."

The total concentration of the three components in refrigerant 1 is preferably 99.7 mass% or more, more preferably 99.8 mass% or more, and even more preferably 99.9 mass% or more, based on the entire composition.

It is particularly preferable that refrigerant 1 consists of HFO-1132(E), HFC-143a, and an amine. However, the presence of unavoidable impurities is acceptable.

Although use of refrigerant 1 is not limited, refrigerant 1 can be used in, for example, refrigerants for air conditioning, heat transfer mediums, or refrigerants for car air conditioners.

### Composition Containing Purified HFO-1132(E)

The present disclosure encompasses a composition containing HFO-1132(E) and an amine ("composition 1" below) as a composition containing purified HFO-1132(E). The type of the amine is as described in the extraction solvent above mentioned.

Composition 1 is a composition containing HFO-1132(E) and an amine, wherein the total concentration of the two components is 99.5 mass% or more based on the entire composition, and the content of the amine is more than 0 mass% and 0.1 mass% or less based on the entire composition. Composition 1 is a composition containing a trace amount of an extraction solvent (amine), among the compositions containing purified HFO-1132 (E) ultimately obtained according to the production method of the present disclosure. The phrase "more than 0 mass%" can also be described as "0.01 mass% or more."

The total concentration of the two components in composition 1 is preferably 99.7 mass% or more, more preferably 99.8 mass% or more, and even more preferably 99.9 mass% or more based on composition 1.

It is particularly preferable that composition 1 consists of HFO-1132(E) and an amine. However, the presence of unavoidable impurities is acceptable.

Although use of composition 1 is not limited, composition 1 can be used in, for example, leak-detecting agents, stabilizers, or tracers during the use of a refrigerant.

### Examples

Examples are provided below to specifically describe the present disclosure. However, the present disclosure is not limited to these Examples.

In the Examples, the concentration of each component, such as HFO-1132(E), was measured with the following measurement device under the following measurement conditions.

Measurement device: gas chromatography (using an FID detector) Method for calculating the concentration of each component:
- Concentration of HFO-1132(E) = number of moles of HFO-1132(E)/(number of moles of HFO-1132(E) + number of moles of HFC-143a)
- Concentration of HFC-143a = number of moles of HFC-143a/(number of moles of HFO-1132(E) + number of moles of HFC-143a)

### Examples 1 to 4 and Comparative Examples 1 to 6

To a composition containing 80 mol% of HFO-1132(E) and 20 mol% of HFC-143a, individual solvents were added in an amount of 5-fold mol based on the composition, and each mixture was measured for specific volatility α_{143a→1132(E)} of HFC-143a to HFO-1132(E) at 15°C.

In Example 1, the solvent for use was monomethylamine, and the specific volatility α_{143a→1132(E)} was 1.7. In Example 2, the solvent for use was monoethylamine, and the specific volatility α_{143a→1132(E)} was 1.8. In Example 3, the solvent for use was mono-n-propylamine, and the specific volatility α_{143a→1132(E)} was 1.6. In Example 4, the solvent for use was mono-isopropylamine, and the specific volatility α_{143a→1132(E)} was 1.7. In Comparative Example 1, the solvent for use was methanol, and the specific volatility α_{143a →1132(E)} was 1.1. In Comparative Example 2, the solvent for use was diethyl ether, and the specific volatility α_{143a→1132(E)} was 1.2. In Comparative Example 3, the solvent for use was n-hexane, and the specific volatility α_{143a→1132(E)} was 1.1. In Comparative Example 4, the solvent for use was methyl isobutyl ketone (MIBK), and the specific volatility α_{143a→1132(E)} was 1.2. In Comparative Example 5, the solvent for use was acetonitrile, and the specific volatility α_{143a→1132(E)} was 1.1. In Comparative Example 6, the solvent for use was ethyl acetate, and the specific volatility α_{143a→1132(E)} was 1.2.

The following summarizes the results.

**Table 2**

| | Solvent | Specific Volatility α_{143a→1132(E)} |
|---|---|---|
| Example 1 | Monomethylamine | 1.7 |
| Example 2 | Monoethylamine | 1.8 |
| Example 3 | Mono-n-propylamine | 1.6 |
| Example 4 | Mono-isopropylamine | 1.7 |
| Comparative Example 1 | Methanol | 1.1 |
| Comparative Example 2 | Diethyl Ether | 1.2 |
| Comparative Example 3 | n-Hexane | 1.1 |
| Comparative Example 4 | Methyl Isobutyl Ketone(MIBK) | 1.2 |
| Comparative Example 5 | Acetonitrile | 1.1 |
| Comparative Example 6 | Ethyl Acetate | 1.2 |

From the results, amines such as monomethylamine, monoethylamine, mono-n-propylamine, and mono-isopropylamine were found to be effective as an extraction solvent.

### Example 5

According to the flow diagram shown in Fig. 1, purified HFO-1132(E) was produced by using an extractive distillation composition containing HFO-1132(E) and HFC-143a as a raw material and mono-isopropylamine as an extraction solvent according to a process consisting of the extractive distillation step and the extraction solvent recovery step.

### Extractive Distillation Step

An extractive distillation composition was supplied at a flow rate of 9.8 mol/hr to an extractive distillation column with 50 theoretical plates from the top of the extractive distillation column through the 15th stage. An extraction solvent (mono-isopropylamine) was supplied from the 4th stage of the extractive distillation column at a flow rate of 206 mol/hr. The operating pressure of the extractive distillation column was 0.45 MPaG, and the column top temperature was -12°C.

### Extraction Solvent Recovery Step

The bottom product extracted from the column bottom in the extractive distillation step was fed from the top of the extraction solvent recovery column with 30 theoretical plates ("solvent recovery column" below) through the 15th stage. A HFO-1132(E) product was recovered from the top of the column with a purity of 99.99%. The operating pressure of the solvent recovery column was 0.6 MPaG, and the column top temperature was -5°C.

The bottom product containing mono-isopropylamine extracted from the bottom of the recovery column (flow rate of mono-isopropylamine: 206 mol/hr) was recirculated to the extractive distillation step and used.

The material balance of Example 5 according to the process in Fig. 1 was the following. In F1, HFO-1132 (E) was 9.4 mol/hr, HFC-143a was 0.4 mol/hr, and mono-isopropylamine was 0 mol/hr. In F2, HFO-1132 (E) was 9.5 mol/hr, HFC-143a was 6 × 10⁻⁵ mol/hr, and mono-isopropylamine was 206.0 mol/hr. In F3, HFO-1132(E) was 2 × 10⁻⁵ mol/hr, HFC-143a was 0.4 mol/hr, and mono-isopropylamine was 2 × 10⁻⁶mol/hr. In F4, HFO-1132 (E) was 0.1 mol/hr, HFC-143a was 6 × 10⁻⁵ mol/hr, and mono-isopropylamine was 206.0 mol/hr. In F5, HFO-1132(E) was 9.4 mol/hr, HFC-143a was 6 × 10⁻⁵ mol/hr, and mono-isopropylamine was 2 × 10⁻¹⁰ mol/hr. Table 3 below summarizes the material balance of Example 5.

**Table 3**

| | F1 | F2 | F3 | F4 | F5 |
|---|---|---|---|---|---|
| HFO-1132 (E) | 9.4 | 9.5 | 2×10⁻⁵ | 0.1 | 9.4 |
| HFC-143a | 0.4 | 6×10⁻⁵ | 0.4 | 6×10⁻⁵ | 6×10⁻⁵ |
| Mono-isopropylamine | 0 | 206.0 | 2×10⁻⁶ | 206.0 | 2×10⁻¹⁰ |
| Unit: mol/hr | | | | | |

The solvents containing amines shown in Examples 1 to 4 all had a specific volatility α_{143a→1132(E)} of HFC-143a to HFO-1132(E) of 1.6 to 1.8, which was sufficiently larger than that of any Comparative Example (1.2 or less), meaning a higher HFC-143a concentration in a gas phase by more than 50%; this indicates a notably improved separation efficiency. Therefore, the process of the present disclosure using a solvent containing an amine in extractive distillation is very effective in the separation of HFC-143a and HFO-1132(E).

### Description of the Reference Numerals

1. Extractive distillation column
2. Solvent recovery column

## Claims

1. A method for producing purified HFO-1132(E), comprising an extractive distillation step of bringing a composition containing 1,1,1-trifluoroethane (HFC-143a) and trans-1,2-difluoroethylene (HFO-1132(E)) into contact with a solvent containing an amine to obtain a composition having a reduced amount of HFC-143a compared with the original composition.

2. The method according to claim 1, further comprising a distillation step of distilling the composition obtained in the extractive distillation step to separate the composition into a composition containing HFO-1132(E) as a main component and a composition containing the solvent containing an amine as a main component.

3. The method according to claim 1, wherein the extractive distillation step comprises extractive distillation performed under a pressure of 0.05 to 5 MPaG (gauge pressure) using a first distillation column.

4. The method according to claim 2, wherein the distillation step comprises distillation performed under a pressure of 0.05 to 3 MPaG (gauge pressure) using a second distillation column.

5. The method according to claim 1, further comprising a solvent recovery step of recovering the solvent containing an amine used in the extractive distillation step, and recirculating the recovered solvent containing an amine to the extractive distillation step.

6. The method according to claim 1, wherein the amine is represented by formula NR¹R²R³
wherein R¹, R², and R³ may be the same or different, and represent a hydrogen atom or an optionally substituted C₁₋₃ hydrocarbon group, with the proviso that not all of R¹, R², and R³ are a hydrogen atom.

7. The method according to claim 1, wherein the amine has a standard boiling point of -10 to 160°C.

8. The method according to claim 1, wherein the amine is at least one member selected from the group consisting of monomethylamine, dimethylamine, trimethylamine, monoethylamine, diethylamine, triethylamine, mono-n-propylamine, di-n-propylamine, tri-n-propylamine, mono-isopropylamine, and di-isopropylamine.

9. The method according to claim 1, further comprising
a distillation step of distilling the composition obtained in the extractive distillation step to separate the composition into a composition containing HFO-1132(E) as a main component and a composition containing the solvent containing an amine as a main component,
wherein
the extractive distillation step comprises extractive distillation performed under a pressure of 0.05 to 5 MPaG (gauge pressure) using a first distillation column, and
the distillation step comprises distillation performed under a pressure of 0.05 to 3 MPaG (gauge pressure) using a second distillation column,
the method further comprising
a solvent recovery step of recovering the solvent containing an amine used in the extractive distillation step, and recirculating the recovered solvent containing an amine to the extractive distillation step, wherein
the amine is at least one member selected from the group consisting of monomethylamine, dimethylamine, trimethylamine, monoethylamine, diethylamine, triethylamine, mono-n-propylamine, di-n-propylamine, tri-n-propylamine, mono-isopropylamine, and di-isopropylamine.

10. A composition comprising trans-1,2-difluoroethylene (HFO-1132(E)), 1,1,1-trifluoroethane (HFC-143a), and an amine, wherein
the total concentration of the three components is 99.5 mass% or more based on the entire composition,
the content of HFC143a is more than 0 mass% and 0.4 mass% or less based on the entire composition, and
the content of the amine is more than 0 mass% and 0.1 mass% or less based on the entire composition.

11. A composition comprising trans-1,2-difluoroethylene (HFO-1132(E)) and an amine,
wherein
the total concentration of the two components is 99.5 mass% or more based on the entire composition, and
the content of the amine is more than 0 mass% and 0.1 mass% or less based on the entire composition.
